# EUROPEAN PATENT APPLICATION

(11) **EP 1 081 235 A2**
(43) Date of publication of application: **07.03.2001**
(21) Application number: 00114992.1
(22) Date of filing: 21.07.2000
(51) Int. Cl.: C14B 1/28, C14B 1/40

(54) **Method and device for measuring the elasticity of hides**

(30) Priority: 02.09.1999 IT BO990474
(71) Applicant: ENEA - ENTE PER LE NUOVE TECNOLOGIE, L'ENERGIA E L'AMBIENTE, 00196 Roma (IT)
(72) Inventor: Rappini, Ruggero, 40125 Bologna (IT)
(74) Representative: Modiano, Guido, Dr.-Ing.

(57) **Abstract**

A method for the automatic nondestructive mapping of the elasticity of hides for manufacturing use, which comprises dividing the entire hide (A) into a plurality of contiguous bands (B) and each one of the bands (B) into a plurality of small regions (C) having a small surface, and subjecting the small regions (C) to a deformation below the assured elasticity limit, measuring the extent of the deformation and of the deforming force applied, processing the measurements of the extent of the deformation and of the deforming force, and recording the set of data computed from the measurements of the extent of the deformation and of the deforming force.

The apparatus for carrying out the method.

## Description

The present invention relates to a method and an apparatus for the automatic nondestructive mapping of the elasticity of hides for manufacturing use.

It is known that in the manufacturing industry's evaluation of the qualitative characteristics of hides it is very important to identify their defects in order to avoid, during cutting, leaving them at least in the exposed regions of the final product. It is also equally important to check their elasticity, i.e., their resistance to traction, as the main parameter linked to the typical stresses produced during the characteristic steps of manufacturing. These stresses can be traced back to deformation actions which substantially belong to two different categories according to their geometry. A first category considers planar unidirectional deformation actions, i.e., deformation actions occurring longitudinally to the surface of the hide (measurement of tensile strength, percent elongation caused by specified load and percent elongation at break according to the IUP/6 international standard), whereas the second category considers deformation actions having a circular symmetry, so-called dome-like deformations (measurement of distension and strength of grain by the ball burst test according to the IUP/9 international standard).

The above types of stress become particularly important in the manufacture of shoes, which carefully assesses the IUP/6 test in relation to the assembly of the sides of the shoe and the IUP/9 test in relation to the forming of the toe.

Complete knowledge of the localized defects and of the elasticity of the hide is an indispensable requirement for manufacturers, in order to assess the workability of the hide, the percentage of use and therefore the possibility to estimate the cost of the finished product.

Currently, the evaluation of the qualities and defects of hides is entrusted to highly qualified personnel. Sometimes the plant manager or the owner personally selects the various batches of hides at the supplying tannery.

In any case, an expert of the manufacturing plant then inspects each individual hide of the batch and points out, before proceeding with the cutting operation, the localized defects and the regions having insufficient elasticity.

Although this method is indispensable in order to have adequate control over the quality of the finished product, it is evidently very onerous in view of the particular specialization required, which is difficult to achieve since it can be acquired only through long practice.

It should also be noted that there is a problem linked to the determination of the quality and price of the hides in the financial and contractual context that involves the supplier and the client which are, usually, the tannery and the manufacturer.

Currently, in case of controversial assessments of the quality of the hides and in legal disputes between the parties, it is customary to resort to the IUP international standards, which provide for destructive testing on the hides at issue, such as the above cited IUP/6 and IUP/9 tests.

Unfortunately, said tests, being destructive on individual specimens, are still inadequate to describe the characteristics of the hide as a whole. The supplying tannery, despite having performed an IUP standardized test on each hide (carried out with a specimen cut from a specifically defined region of said hide), in fact is often unable to certify each hide entirely, since as is well-known each hide inherently has regions with highly uneven characteristics.

On the other hand, the manufacturing customer has the evident need to maximize utilization of the hides that he purchases, and would like a guarantee that said quality characteristics of the product are known in all of its types, over the entire extension of the hide, and are not below an agreed standard for the negotiated price.

The above description clearly shows the urgent need to correct the above described inconvenience and the frequent legal disputes arising therefrom, not due to the ill will of the supplier but rather due to an inherent inadequacy of the current methods, typically based on destructive tests.

The aim of the present invention is to obviate the above drawbacks of conventional methods and tests, by providing a method and an apparatus capable of detecting and recording the physical and mechanical characteristics of the various categories of hides used without damaging them, and also capable of describing the entire hide in relation to its workability characteristics, such as elasticity or resistance to traction, to ensure a finished product having the necessary and constant quality.

Within the scope of this aim, an object of the present invention is to use said apparatus and method in fields similar to the manufacturing sector, working on other kinds of material, such as for example fabrics and laminates which can also be used in the manufacturing field of shoes, leather goods and clothes.

Within the scope of this aim, another object of the present invention is that of providing a method and an apparatus for evaluating hides, which are simple, relatively easy to actuate in practice, effective in operation, and at a low cost.

These and other objects are achieved by a method, according to the invention, for the automatic nondestructive mapping of the elasticity of hides for manufacturing use, characterized in that it comprises dividing the entire hide into a plurality of contiguous bands, and each one of said bands into a plurality of small regions having a small surface, subjecting said small regions to a deformation which is below the assured elasticity limit, measuring the extent of said deformation and the deforming force applied, processing the measurements of the extent of said deformation and of said deforming force, and recording a set of data computed from the measurements of the extent of said deformation and of said deforming force.

An apparatus for performing the method, according to the invention, comprises: means for locking said hide so as to form at least one acquisition band which can be divided into said small regions; means for pushing probes which are adapted to engage against said hide with a force which can be preset and is such that the deformation of the hide that it produces is within the limit of assured elasticity; means for measuring the extent of the deforming force applied by said probes to said small regions of the hide; means for measuring the extent of the deformation of said small regions; means for computing the measurements of the extent of said deformation and of said measured deforming force; and means for recording a set of computed data related to the extent of the deformations and of the deforming forces applied to said bands and said small regions.

Further features will become better apparent from the detailed description of a preferred but not exclusive embodiment of a method and an apparatus for the automatic nondestructive mapping of the elasticity of hides for manufacturing use, according to the invention, illustrated only by way of non-limitative example in the accompanying drawings, wherein:

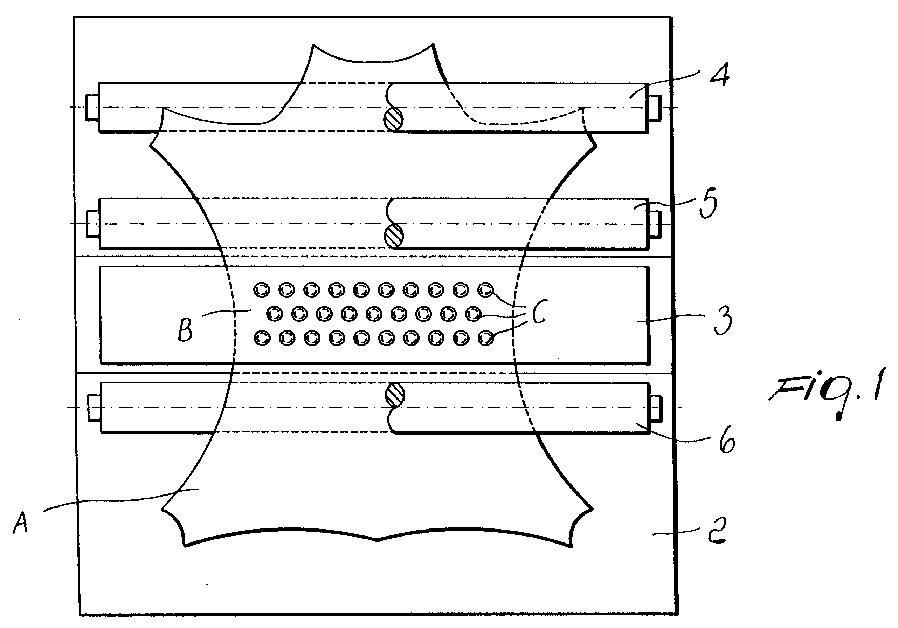

Figure 1 is a plan view of the hide A subjected to the method according to the invention by means of the apparatus for automatically mapping the elasticity of hides;

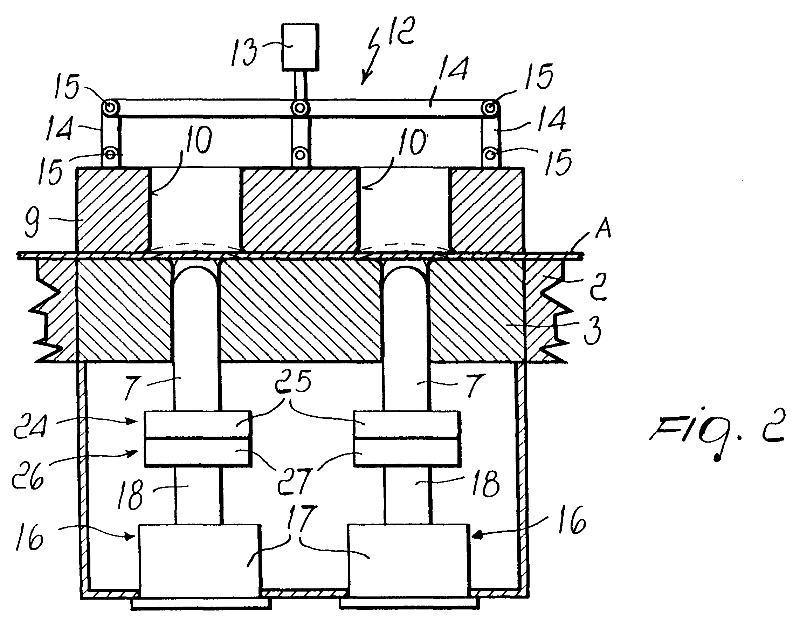

Figure 2 is a sectional view, taken along a plane which is perpendicular to the apparatus according to the invention;

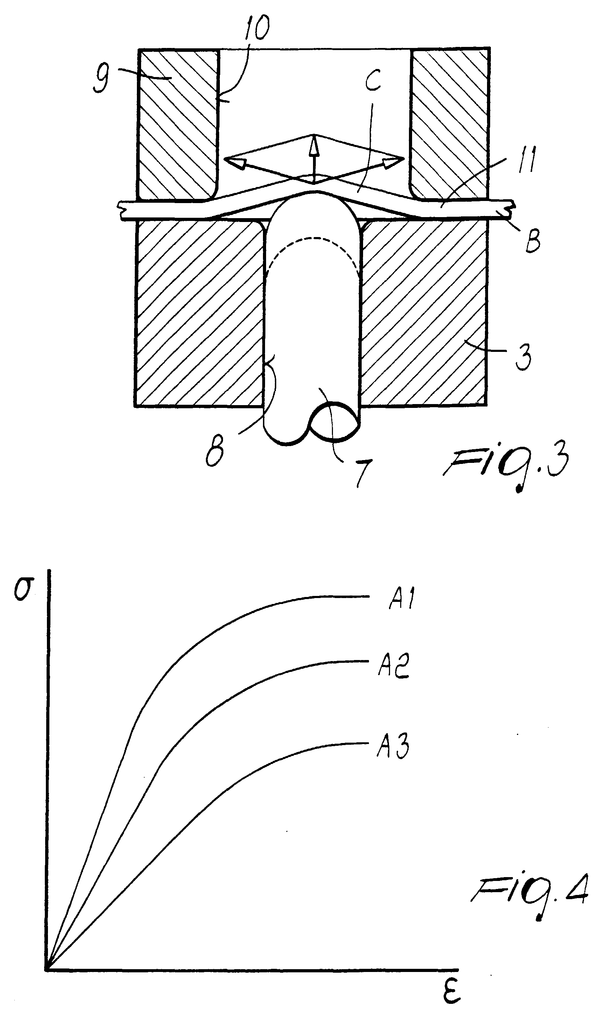

Figure 3 is a sectional view of a detail of a movable probe which is adapted to map hides;
Figure 4 is a chart of a traction test on three different types of hide A1, A2, and A3, having a different elastic behavior;
Figure 5 is a view of a further embodiment of the plate and the contrast plate of the apparatus according to the invention;
Figure 6 is a view of a further embodiment of the pusher means of the movable probes of the apparatus according to the invention;
Figure 7 is a view of a first embodiment of the movable probes of the apparatus according to the invention;
Figure 8 is a view of a third embodiment of the roller-type movable probes of the apparatus according to the invention;
Figure 9 is a view of a second embodiment of the movable probes of the apparatus according to the invention;
Figure 10 is a view of a fourth embodiment of the movable probes of the apparatus according to the invention, shaped as laminae;
Figure 11 is a diagram of the circuit connections between the parts of the apparatus according to the invention.

With reference to the above figures, the letter A generally designates a hide for automatic mapping. The hide is divided into a plurality of contiguous bands B, and each one of said bands is divided into a plurality of small regions C having a small surface. The small regions C are subjected to a deformation which is kept within the limit of the assured elasticity of the hide (Figure 4). This means that in each instance, according to the type of hide A1, A2, A3 subjected to mapping, the deformation and the extent of the deforming force applied are preset to different values, such as to avoid damaging the hide being examined. The resulting measurements are processed so as to correct any systematic or approximation errors. The mechanical characteristics that cannot be measured directly, such as for example the elastic modulus of the hide A at the small regions C, are also computed. Computation is entrusted to an electronic computer, which sends a set of the computed data to an identification and memory element. Said set of computed data of the measurements of the extent of the deformation and of the deforming force is recorded on the identification and memory element, which in turn is fixed to the hide A as it leaves the present mapping process.

With reference to the above figures, 1 generally designates an apparatus for automatically mapping the hide. The apparatus 1 is composed of a worktable 2 provided with a flat plate 3 arranged in a central position. The hide A to be mapped is made to slide on the plate 3 by means of first, second and third pairs of cylinders 4, 5 and 6. The pairs of cylinders 4, 5 and 6 are arranged on the opposite sides of the plate 3, respectively with the pairs 4,5 on one side and the pair 6 on the other side. The pairs 4, 5 are driving pairs and the pair 6 is braked, so that the band of hide B that lies between the pairs 5 and 6 is tensioned with a presettable force.

The movable probes 7 engage against the band of hide B and are guided in a plurality of holes 8 of the flat plate 3. The holes 8 are aligned on three staggered rows, so as to uniformly distribute the small regions C in which the characteristics of the hide A are measured.

The hide locking contrast plate 9 is movable towards and away from the plate 3 and has a plurality of openings 10 which are suitably larger than, and substantially aligned with, the holes 8; a space 11 for the sliding of the hide A is formed between the plate 3 and the contrast plate 9. The contrast plate 9 is moved by means 12 for approach and spacing. The means 12 for approach and spacing at preset pressure between the plate 3 and said contrast plate 9 comprise an actuator 13 of the type that operates by means of a pressurized fluid and is kinematically connected to a series of rigid rods 14 and articulations 15 for connecting the actuator 13 to the contrast plate 9. Said approach means compress the band of hide B between the plate 3 and the contrast plate 9 in the regions between the acquisition regions C. The pressure with which the actuator 13 acts on the contrast plate is therefore preset according to the type of hide A1, A2, A3 being examined.

The movable probes 7 are actuated by pusher means 16 which comprise a piston 17 which acts on the lower portion 18 of the movable probe 7 and can be actuated by means of a circuit 19 which contains a fluid. The circuit 19 is composed of a duct 20 for connection between the piston 17 and a pump 21 for compressing the fluid, by a pressure measuring instrument 22 and by a valve 23 for adjusting and controlling the pressure in the duct 20. The movable probes 7 are used to map the hide according to two main modes. The first mode applies to the hide a known stress and then measures the extent of the resulting deformation. The second mode applies a known deformation and then measures the extent of the force with which the hide reacts to said known deformation.

The means for measuring the extent of the deformation 24 of the acquisition band B and of the small regions C comprise a plurality of sensors 25 for detecting the relative movement between the plate 3 and the probes 7.

The means for measuring the extent of the deforming force 26 applied to the hide by the probes 7 comprise a plurality of sensors 27 of the load-cell type for measuring the force generated between the probes 7 and the hide A.

The measurements acquired by said measurement means 24 and 26 are transmitted to the processing means 28. The processing means comprise an electronic computer 29 which is circuitally connected both to the measurement means 24 and 26 and to recording means 30.

The recording means 30 are constituted by an identification and memory element in which the set of data processed by the computer 29 can be stored and read. The set of processed data comprises the measurements obtained by virtue of the measurement means 24 and 26 and corrected appropriately, and the characteristics of the hide that cannot be measured directly, such as for example the elastic modulus obtained, as shown in Figure 4, from the ratio between the tension σ and the deformation ε, the tension σ being in turn determinable as a function of the applied deforming force. The identification and memory element can be fixed onto each hide A subjected to the mapping process and is constituted by a memory microchip of the RAM (random access memory) type which is adapted to be read, written and rewritten, being recoverable and reusable.

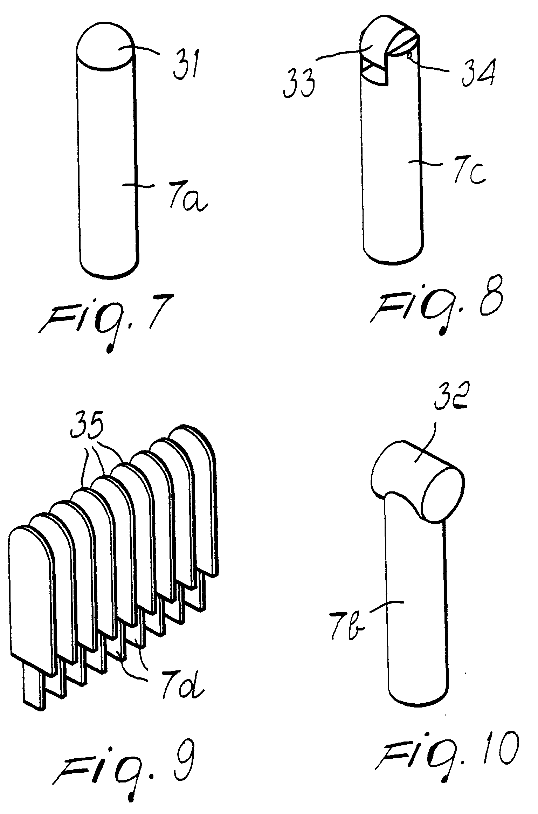

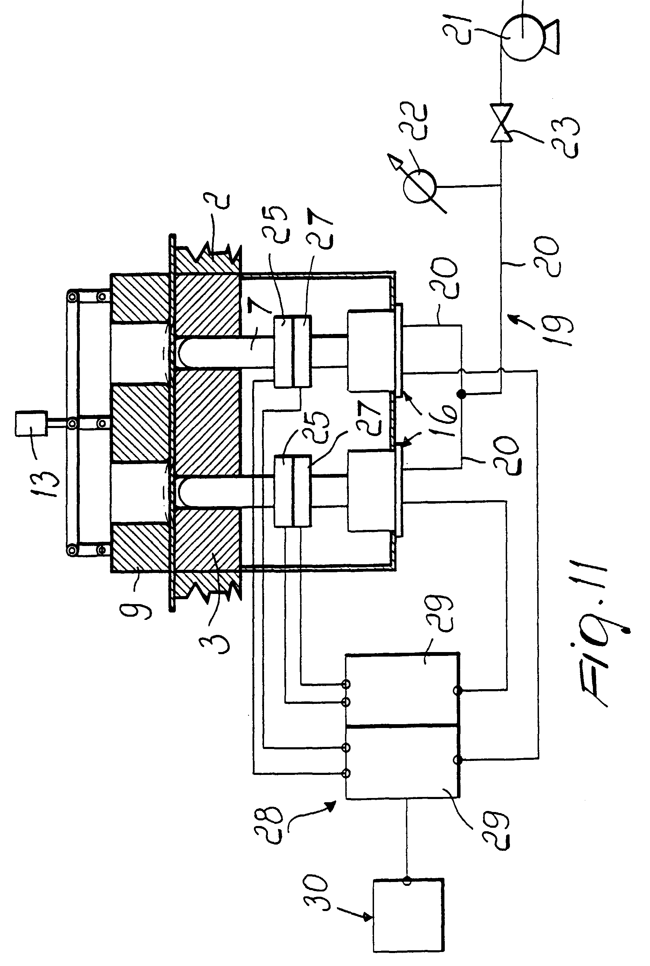

The apparatus 1 further provides for the possibility to use movable probes having different shapes. The first embodiment, shown in Figure 7, uses a movable probe 7a in which the end portion 31, adapted to make contact with the hide, is substantially hemispherical. The second embodiment, shown in Figure 10, uses a movable probe 7b in which the end portion 32, adapted to make contact with the hide, is substantially cylindrical. In a third embodiment, shown in Figure 8, the movable probe 7c uses a roller 33 which can turn freely about a pivot 34 which is supported at the top of the probe 7c. The fourth embodiment of a movable probe 7d is shown in Figure 9 and provides for a plurality of laminae 35 which are arranged side by side and can be actuated individually or in presettable groups.

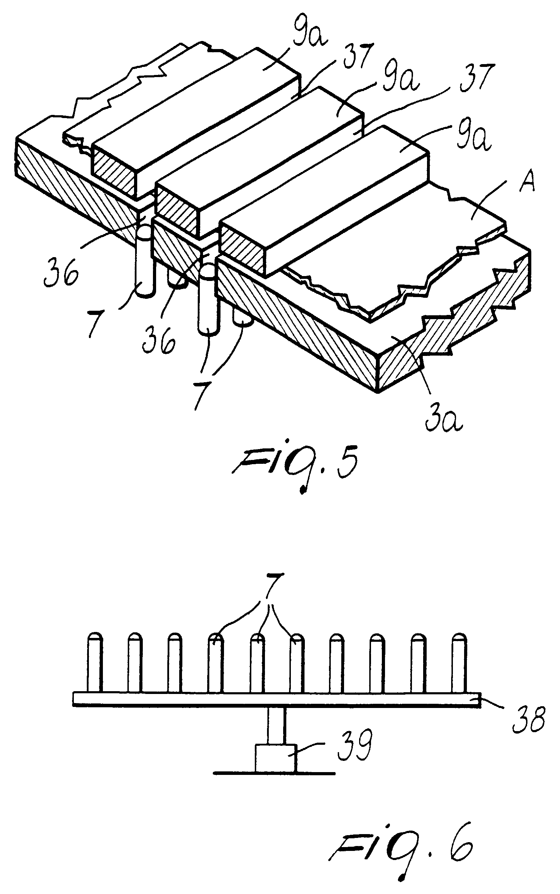

It is noted that if the shapes of the movable probes 7 vary, it is necessary to also vary the geometry of the plate 3 and of the contrast plate 9, which are shown in an embodiment having a different shape 3a and 9a in Figure 5. In this embodiment, the flat plate 3a is crossed by a plurality of first parallel slots 36 which accommodate roller movable probes 7c or lamina movable probes 7d. The contrast plate 9a has a second plurality of slots 37 which have an appropriately larger gap and are substantially aligned with the first slots 36. Moreover, the means 16 for pushing the movable probes also have an embodiment 16a, shown in Figure 6. Said figure shows that the movable probes 7 can be actuated simultaneously by means of a horizontal connecting bar 38 which can raise and lower the probes in rows, being moved by a hydraulic piston 39.

The roller movable probes 7c can be used without the aid of the contrast plate 9, since they can operate merely thanks to the advancement at a presettable speed imparted by the pairs of cylinders 4, 5 and 6. A dynamic locking of the band of hide B is in fact provided thanks to the simple traction applied to the hide by the combined action of the pairs of driving cylinders 4, 5 and the pair of braked cylinders 6.

Another embodiment of the present apparatus is the use of a different identification and memory element, which also can be fixed to each hide A subjected to the mapping process and is constituted by a movable radio-frequency tag which is adapted to be read, written and rewritten, being recoverable and reusable.

The operation of the apparatus 1 is discontinuous if the movable probes 7a, 7b and 7d and the contrast plate 9 or 9a are used. The same mechanical characteristics that distinguish the IUP/9 test can be acquired by using the probes 7a, while the IUP/6 test is simulated by means of the probes 7b and 7d.

The operation of the apparatus 1 is continuous when the roller movable probes 7c are used, with or without the contrast plate 9a. Said probes simulate the IUP/6 test and considerably increase the speed with which the entire mapping process occurs.

It has thus been shown that the invention achieves the intended aim and objects.

The invention thus conceived is susceptible of numerous modifications and variations, all of which are within the scope of the appended claims.

All the details may furthermore be replaced with other technically equivalent ones.

In practice, the materials used, as well as the shapes and the dimensions, may be any according to requirements without thereby abandoning the protective scope of the appended claims.

The disclosures in Italian Patent Application No. BO99A000474 from which this application claims priority are incorporated herein by reference.

Where technical features mentioned in any claim are followed by reference signs, those reference signs have been included for the sole purpose of increasing the intelligibility of the claims and accordingly, such reference signs do not have any limiting effect on the interpretation of each element identified by way of example by such reference signs.

## Claims

1. A method for the automatic nondestructive mapping of the elasticity of hides for manufacturing use, characterized in that it comprises the steps of: dividing the entire hide (A) into a plurality of contiguous bands (B) and each one of said bands into a plurality of small regions (C) having a small surface; subjecting said small regions (C) to a deformation which is below the assured elasticity limit of the hide; measuring the extent of said deformation and the extent of the deforming force applied; processing the measurements of the extent of said deformation and of said deforming force; and recording a set of data computed from the measurements of the extent of said deformation and of said deforming force.

2. The apparatus for performing the method according to claim 1, characterized in that it comprises: means (3,9) for locking said hide (A) so as to form at least one acquisition band (B) which can be divided into said small regions (C); means (16) for pushing movable probes (7) which are adapted to engage against said hide (A) with a force which can be programmed and is such that the deformation of the hide (A) that it produces is within the limit of assured elasticity, said probes (7) being also adapted to engage against said hide (A), producing a programmable deformation; means for measuring the extent of the deforming force (26) applied by said probes (7) against said small regions (C) of the hide (A); means for measuring the extent of the deformation (24) of said small regions (C); means for processing (28) the acquired measurements of the extent of said deformation and of said deforming force; means for recording (30) the set of processed data of the extent of the deformations and of the deforming forces which are applied to said bands (B) and said small regions (C).

3. The apparatus according to one or more of the preceding claims, characterized in that said means (3,9) for locking said hide comprise a flat plate (3) which is crossed by a plurality of holes (8) for the guided sliding of said hide movable probes (7), a contrast plate (9) for locking the hide (A) which can be moved toward and away from said plate (3) and is provided with a plurality of openings (10) which are conveniently larger than said holes (8) and are substantially aligned with them, a space (11) for the sliding of said hide (A) being formed between said plate (3) and said contrast plate (9), means (12) for the approach and spacing at a preset pressure of said plate (3) and said contrast plate (9) being also provided.

4. The apparatus according to one or more of the preceding claims, characterized in that said means for locking (3,9) said hide comprise a flat plate (3a) crossed by a plurality of first parallel slots (36) which accommodate roller movable probes (7c) which are adapted to engage against said small regions (C) of hide (A), a contrast plate (9a) which can be moved towards and away from said plate (3a) and is provided with a second plurality of slots (37) having an appropriately larger gap and being substantially aligned with said first slots (36), a space for the transverse sliding of said hide (A) with respect to the arrangement of said first slots (36) being formed between said plate (3a) and said contrast plate (9a), means for the approach and spacing at a preset pressure of said plate (3a) and said contrast plate (9a) being also provided.

5. The apparatus according to one or more of the preceding claims, characterized in that said means (3,9) for locking said hide comprise a flat plate (3a) crossed by a plurality of first parallel slots (36) which accommodate roller movable probes (7c) which are adapted to engage against said small regions (C) of hide (A), at least one first and second pairs (4,5) and one third pair (6) of cylinders arranged parallel to each other, respectively on either side of said plate (3a), and adapted to provide the advancement, at a preset speed, and the dynamic locking of said band (B) of hide, said first and second pairs (4,5) of cylinders being driving pairs, said third pair (6) of cylinders being a braked pair.

6. The apparatus according to one or more of the preceding claims, characterized in that said means (12) for moving said plate (3) and said contrast plate (9) closer and further apart with a preset pressure comprise at least one actuator (13) operating by way of a pressurized fluid and kinematically connected to a plurality of articulations (15) of said contrast plate (9).

7. The apparatus according to one or more of the preceding claims, characterized in that each one of said pusher means (16) for the movable probes (7) comprises a piston (17) acting on the lower portion of the respective probe (7) and being actuated by way of a circuit (19) which contains a fluid and is composed of at least one duct (20) for connection between said piston (17) and a fluid compression pump (21), by an instrument (22) for measuring said pressure and by a valve (23) for adjusting and controlling the pressure in said duct (20).

8. The apparatus according to one or more of the preceding claims, characterized in that said means for measuring the extent of the deformation (24) of said acquisition band (B) and of said small regions (C) comprise a plurality of sensors (25) for detecting the relative displacement between said plate (3) and said probes (7).

9. The apparatus according to one or more of the preceding claims, characterized in that means for measuring the extent of the deforming force (26) applied to the hide (A) by said probes (7) comprise a plurality of sensors (27) of the load-cell type for detecting the force that is generated between said probes (7) and said hide (A).

10. The apparatus according to one or more of the preceding claims, characterized in that said processing means (28) comprise an electronic computer (29) circuitally connected to said measurement means (24,26) and to said recording means (30).

11. The apparatus according to one or more of the preceding claims, characterized in that said recording means (30) are constituted by an identification and memory element in which it is possible to store and read said set of processed data, said identification and memory element being fixable onto each hide (A) subjected to said mapping process.

12. The apparatus according to one or more of the preceding claims, characterized in that said identification and memory element comprises at least one memory microchip of the RAM (random access memory) type and is adapted to be read, written and rewritten, being recoverable and reusable.

13. The apparatus according to one or more of the preceding claims, characterized in that said identification and memory element comprises a movable radio-frequency tag and is adapted to be read, written and rewritten, being recoverable and reusable.

14. The apparatus according to one or more of the preceding claims, characterized in that said movable probes (7a) comprise an end portion (31), adapted to make contact with the hide (A), which has a substantially hemispherical shape.

15. The apparatus according to one or more of the preceding claims, characterized in that said movable probes (7b) comprise an end portion (32), adapted to make contact with the hide (A), which has a substantially cylindrical shape.

16. The apparatus according to one or more of the preceding claims, characterized in that said roller movable probes (7c) comprise an upper portion (33) which is in contact with the hide (A), is substantially cylindrical, is supported (34) so that it can rotate freely and is adapted to rotate against said hide (A).

17. The apparatus according to one or more of the preceding claims, characterized in that said movable probes (7d) comprise a plurality of side-by-side laminae (35) which can be actuated individually or in preselectable groups.
